# EUROPEAN PATENT SPECIFICATION

(11) **EP 2 091 500 B1**
(45) Date of publication and mention of the grant of the patent: **23.10.2013**
(21) Application number: 07865575.0
(22) Date of filing: 12.12.2007
(51) Int. Cl.: A61K 8/02, A61Q 19/00, C11D 3/22, C11D 17/00

(54) **SURFACTANT THICKENED SYSTEMS COMPRISING MICROFIBROUS CELLULOSE AND METHODS OF MAKING SAME**
VERDICKTE TENSIDSYSTEME MIT MIKROFASERZELLULOSE UND HERSTELLUNGSVERFAHREN
SYSTÈMES ÉPAISSISSANTS TENSIOACTIFS COMPRENANT DE LA CELLULOSE MICROFIBREUSE ET LEURS PROCÉDÉS DE FABRICATION

(30) Priority: 15.12.2006 US 611492
(43) Date of publication of application: 26.08.2009
(73) Proprietor: CP Kelco US, Inc., Atlanta, GA 30339 (US)
(72) Inventor: SWAZEY, John M., San Diego, CA 92130 (US)
(74) Representative: D'Arcy, Julia
(86) International application number: PCT/US2007/087229
(87) International publication number: WO 2008/076753

(56) References cited:
- WO-A1-99/40153
- WO-A1-03/085074
- US-A1- 2006 281 859
- US-B1- 6 302 209

## Description

### BACKGROUND OF THE INVENTION

Surfactant-based products such as body washes, shampoos, bubble bath, dish soap, automatic dishwashing detergents, laundry detergents, automotive detergents, toilet cleaners, surfactant concentrates, fire-fighting foaming agents, among others, are often thickened by utilizing high concentration of surfactants, by combining viscosity synergistic surfactants, or by combining the surfactants with small amounts of salts, such as sodium salts. These formulations result in high viscosity products that appear rich and smooth but they are limited in that they do not provide sufficient low shear viscosity to allow for suspension of particles. Such particulates might include aesthetic agents (decorative beads, pearlescents, air bubbles, fragrance beads, etc.) or active ingredients (insoluble enzymes, encapsulated actives such as moisturizers, zeolites, exfoliating agents (e.g. alpha hydroxyl and/or glycolic acids or polyethylene beads), vitamins (e.g. vitamin E)) etc. or both.

Conventional thickeners and suspension aids such as xanthan gum, carboxymethyl cellulose (CMC), hydroxyethylcellulose (HEC), hydroxypropylmethylcellulose (HPMC), and many types of polyacrylates do not function well with high surfactant levels or in surfactant-thickened systems and often lead to a loss of transparency due to clouding, gelling, and/or phase separation or lack sufficient suspension properties, For example, xanthan gum imparts excellent suspension properties in certain body wash formulations with low surfactant-thickening but the gum often loses its suspension ability in systems with high surfactant thickening, usually resulting in a hazy, irregular appearance, and a grainy or lumpy texture. Cellulosic products (CMC, HEC, HPMC, etc.), as another example of conventional thickeners, provide unreliable suspension and have significant limitations with respect to surfactant compatibilities. Acrylates systems are common, however, these systems do not always achieve a sufficient clarity level, require high concentrations of polymer, and are not considered natural. Salts are often capable of increasing high shear viscosity in surfactant-thickened systems but do not impart long-term suspension ability.

There is presently a desire in the consumer products industry to provide for transparent surfactant-thickened systems with particulates suspended therein, as well as a suspension aid for high surfactant systems where many alternative thickeners will not function.

It has been discovered that microfibrous cellulose (MFC), bacterially derived or otherwise, can be used to provide suspension of particulates in surfactant-thickened systems as well as in formulations with high surfactant concentrations. It was also discovered that the MFC may be used for this purpose with or without co-agents. When bacterially-derived microfibrous cellulose is utilized, cellular debris can be eliminated which results in transparent solutions at typical use levels.

The microfibrous cellulose appears unaffected by the surfactant micelle development and maintains good suspension in these systems. Microfibrous cellulose is unique in its ability to function in these systems in large part because it is dispersed rather than solubilized, thereby achieving the desired suspension properties in formulations that would otherwise display the hazing and/or precipitation often seen using alternative solubilized polymers.

### BRIEF SUMMARY OF THE INVENTION

Surfactant systems comprising microfibrous cellulose are described. "Surfactant systems" is intended to include but is not limited to surfactant-thickened and high surfactant systems. Microfibrous cellulose (MFC) includes MFC prepared by microbial fermentation or MFC prepared by mechanically disrupting/altering cereal, wood, or cotton-based cellulose fibers. When bacterially-derived microfibrous cellulose is utilized, cellular debris can be eliminated which results in transparent solutions at typical use levels. The present invention utilizes surfactants to achieve a very thick (highly viscous) system at high shear rates with particulates suspended therein by using microfibrous cellulose.

Industrial surfactant concentrates (for later dilution by manufacturing or the consumer) can have surfactant levels near 100% for non-ionic surfactants, and sometimes over 50% for anionic surfactants. These concentrates can be used in the manufacture of consumer products such as bath soaps and shampoos or for applications such as fire-fighting foams where the surfactant is diluted in use. The MFC can be added to these concentrates to provide yield stress to the concentrate or to the diluted system. The MFC is present at concentrations from about 0.05% to about 1.0%, but the concentration will depend on the desired product For example, while about 0.06% (w/w) MFC is preferred for suspending small air bubbles in an 80% surfactant system, about 0.078% is preferred for suspending air bubbles in a 99% surfactant system. Furthermore, the concentration of MFC will be adjusted accordingly if a highly transparent system is desired.

Particulates to be suspended could include aesthetic agents (decorative beads, pearlescents, air bubbles, fragrance beads, etc.) or active ingredients (insoluble enzymes, encapsulated actives such as moisturizers, zeolites, exfoliating agents (e.g. alpha hydroxyl and/or glycolic acids or polyethylene beads), vitamins (e.g. vitamin E) etc. or both. Other suitable particulates would be apparent to one of skill in the art.

The invention is also directed to the use of co-agents and/or co-processing agents such as CMC, xanthan, and/or guar gum with the microfibrous cellulose in the surfactant systems described herein. Microfibrous cellulose blends are microfibrous cellulose products which contain co-agents. Two blends are described MFC, xanthan gum, and CMC in a ratio of 6:3:1, and MFC, guar gum, and CMC in a ratio of 3:1:1. These blends allow MFC to be prepared as a dry product which can be "activated" with high shear or high extensional mixing into water or other water-based solutions. "Activation" occurs when the MFC blends are added to water and the co-agents/co-processing agents are hydrated. After the hydration of the co-agents/co-processing agents, high shear is generally then needed to effectively disperse the microfibrous cellulose fibers to produce a three-dimensional functional network that exhibits a true yield point Unexpectedly, the co-agent and/or co-processing agents CMC, xanthan, and/or guar gum present in these microfibrous cellulose blends appear to remain solubilized (after activation in water) in many high surfactant formulations despite their general lack of compatibility in the high surfactant systems, most likely due to the low use level of these polymers in these formulations with MFC.

The invention is further directed to methods of making the surfactant systems described, with or without co-agents and/or co-processing agents.

Claims 2 to 9 and 11 to 15 set out preferred embodiments of the invention

### BRIEF DESCRIPTION OF THE SEVERAL VIEWS OF THE DRAWINGS

The foregoing summary will be better understood when read in conjunction with the Detailed Description of the Invention.

### DETAILED DESCRIPTION OF THE INVENTION

Solutions containing high levels of surfactant were prepared using microfibrous cellulose with and without co-agents. The pH of the systems described herein range from about 2 to about 12.

### EXAMPLE 1

A thickened solution containing 80% non-ionic surfactant was prepared with 0.1% microfibrous cellulose blend (MFC/xanthan/CMC 6:3:1 blend). A concentrate was first prepared containing 0.5% microfibrous cellulose blend (MFC/xanthan/CMC 6:3:1 blend) in deionized water. 40 g of this solution was introduced into a 250 ml beaker and then 160 g of undiluted Triton(R) X-100 (100% active Octoxynol-9 from Union Carbide) was added slowly with mixing at 600 rpm using a jiffy mixing blade. The resulting solution exhibited good clarity upon visual inspection and possessed the ability to suspend polyethylene beads, gelatin encapsulates, gellan gum beads, and air bubbles. The yield value was 0.33 Pa (as measured with a Brookfield(R) Yield Rheometer) at a pH of 5.3.

### EXAMPLE 2

A thickened solution containing 80% non-ionic surfactant was prepared with 0.1% microfibrous cellulose blend (MFC/xanthan/CMC 6:3:1 blend). A concentrate was first prepared containing 0.5% microfibrous cellulose blend (MFC/xanthan/CMC 6:3:1 blend) in deionized water. 40 g of this solution was put into a 250 ml beaker and 160 g of undiluted Tween(R) 20 (100% active Polysorbate 20 from ICI) was added slowly with mixing at 600 rpm using a jiffy mixing blade. The resulting solution exhibited good clarity upon visual inspection and possessed the ability to suspend polyethylene beads, gelatin encapsulates, gum arabic encapsulates, and air bubbles. The yield value was 0.11 Pa (as measured with a Brookfield(R) Yield Rheometer) at a pH of 6.0.

### EXAMPLE 3

A thickened solution containing 99% non-ionic surfactant was prepared using a wet-cake version of microfibrous cellulose. 0.78% wet cake was added to undiluted Triton X-100 and mixed on an Oster(R) blender at "liquefy" (top speed) for 5 minutes. The activity (% solids) of this wet-cake form of MFC was about 16% so the active MFC level was 0.125% in the surfactant. The resulting solution exhibited good clarity upon visual inspection and possessed the ability to suspend polyethylene beads, gelatin encapsulates, gum arabic encapsulates, and air bubbles. The solution was de-aerated under vacuum and the yield point was taken. Upon visual inspection the resulting solution exhibited good clarity with a slight haze and a yield point of 14.6 Pa.

### EXAMPLE 4

A thickened solution containing 99% non-ionic surfactant was prepared using the wet-cake version of microfibrous cellulose. 0.78% wet cake was added to undiluted Tween(R) 20 and mixed on an Oster(R) blender at "liquefy" (top speed) for 5 minutes. The activity (% solids) of this wet-cake form of MFC was 16% resulting in an active MFC level of 0.125% in the surfactant. The resulting solution exhibited good clarity upon visual inspection and possessed the ability to suspend polyethylene beads, gelatin encapsulates, gum arabic encapsulates, and air bubbles. The solution was de-aerated under vacuum and the yield point was determined. Upon visual inspection the resulting solution exhibited good clarity with some haze and a yield point of 17.8 Pa.

## Claims

1. A surfactant system comprising microfibrous cellulose at a concentration of about 0.05% to about 1.0% (w/w), surfactant at about 51 % to about 99% (w/w active surfactant), and a suspended particulate.

2. The surfactant system according to claim 1, wherein the microfibrous cellulose is present at about 0.06%.

3. The surfactant system according to claim 1, wherein the microfibrous cellulose is present at about 0.075%.

4. The surfactant system according to claim 1, wherein the microfibrous cellulose is present at about 0.125%.

5. The surfactant system according to claim 2, wherein the surfactant is present at about 80% (w/w active surfactant).

6. The surfactant system according to claim 5, wherein the particulate comprises air bubbles.

7. The surfactant system according to claim 4, wherein the surfactant is present at about 99% (w/w active surfactant).

8. The surfactant system of claim 5 wherein the pH is from about 3 to about 11.

9. The surfactant system of claim 7 wherein the pH is from about 3 to about 11.

10. Method of preparing a surfactant system, comprising,
combining microfibrous cellulose with water and mixing,
adding surfactant and then mixing,
adding particulates followed by mixing,
wherein the resulting system is transparent and the particulates are suspended therein,
wherein the microfibrous cellulose is present at a concentration of about 0.05% to about 1.0% (w/w), and the surfactant is present at about 51 % to about 99% (w/w active surfactant).

11. The method of claim 10 wherein the microfibrous cellulose is present at about 0.06%.

12. The method of claim 10, wherein the microfibrous cellulose is present at about 0.075%.

13. The method of claim 10, wherein the surfactant is present at about 80% (w/w active surfactant).

14. The method of claim 10, wherein the surfactant is present at about 99% (w/w active surfactant).

15. The method of any of claims 10 to 14, wherein the microfibrous cellulose is prepared as a dry product comprising a co-agent.

## Patentansprüche

1. Ein Tensidsystem, das Mikrofaserzellulose mit einer Konzentration von etwa 0,05 % bis etwa 1,0 % (Massenanteil), Tensid mit etwa 51 % bis etwa 99 % (Massenanteil aktives Tensid) und einen suspendierten Partikelstoff beinhaltet.

2. Tensidsystem gemäß Anspruch 1, wobei die Mikrofaserzellulose mit etwa 0,06 % vorhanden ist.

3. Tensidsystem gemäß Anspruch 1, wobei die Mikrofaserzellulose mit etwa 0,075 % vorhanden ist.

4. Tensidsystem gemäß Anspruch 1, wobei die Mikrofaserzellulose mit etwa 0,125 % vorhanden ist.

5. Tensidsystem gemäß Anspruch 2, wobei das Tensid mit etwa 80 % (Massenanteil aktives Tensid) vorhanden ist.

6. Tensidsystem gemäß Anspruch 5, wobei der Partikelstoff Luftblasen beinhaltet.

7. Tensidsystem gemäß Anspruch 4, wobei das Tensid mit etwa 99 % (Massenanteil aktives Tensid) vorhanden ist.

8. Tensidsystem gemäß Anspruch 5, wobei der pH-Wert von etwa 3 bis etwa 11 beträgt.

9. Tensidsystem gemäß Anspruch 7, wobei der pH-Wert von etwa 3 bis etwa 11 beträgt.

10. Ein Verfahren zum Herstellen eines Tensidsystems, das Folgendes beinhaltet:
Kombinieren von Mikrofaserzellulose mit Wasser und Mischen,
Hinzufügen von Tensid und dann Mischen,
Hinzufügen von Partikelstoffen, gefolgt von Mischen,
wobei das resultierende System transparent ist und die Partikelstoffe darin suspendiert sind,
wobei die Mikrofaserzellulose mit einer Konzentration von etwa 0,05 % bis etwa 1,0 % (Massenanteil) vorhanden ist und das Tensid mit etwa 51 % bis etwa 99 % (Massenanteil aktives Tensid) vorhanden ist.

11. Verfahren gemäß Anspruch 10, wobei die Mikrofaserzellulose mit etwa 0,06 % vorhanden ist.

12. Verfahren gemäß Anspruch 10, wobei die Mikrofaserzellulose mit etwa 0,075 % vorhanden ist.

13. Verfahren gemäß Anspruch 10, wobei das Tensid mit etwa 80 % (Massenanteil aktives Tensid) vorhanden ist.

14. Verfahren gemäß Anspruch 10, wobei das Tensid mit etwa 99 % (Massenanteil aktives Tensid) vorhanden ist.

15. Verfahren gemäß einem der Ansprüche 10 bis 14, wobei die Mikrofaserzellulose als Trockenstoff hergestellt wird, der ein Hilfsmittel beinhaltet.

## Revendications

1. Un système tensioactif comprenant de la cellulose microfibreuse à une concentration allant d'environ 0,05 % à environ 1,0 % (p/p), un tensioactif à raison d'environ 51 % à environ 99 % (p/p tensioactif actif), et une matière particulaire en suspension.

2. Le système tensioactif selon la revendication 1, dans lequel la cellulose microfibreuse est présente à raison d'environ 0,06 %.

3. Le système tensioactif selon la revendication 1, dans lequel la cellulose microfibreuse est présente à raison d'environ 0,075 %.

4. Le système tensioactif selon la revendication 1, dans lequel la cellulose microfibreuse est présente à raison d'environ 0,125 %.

5. Le système tensioactif selon la revendication 2, dans lequel le tensioactif est présent à raison d'environ 80 % (p/p tensioactif actif).

6. Le système tensioactif selon la revendication 5, dans lequel la matière particulaire comprend des bulles d'air.

7. Le système tensioactif selon la revendication 4, dans lequel le tensioactif est présent à raison d'environ 99 % (p/p tensioactif actif).

8. Le système tensioactif de la revendication 5 dans lequel le pH va d'environ 3 à environ 11.

9. Le système tensioactif de la revendication 7 dans lequel le pH va d'environ 3 à environ 11.

10. Méthode de préparation d'un système tensioactif, comprenant,
combiner de la cellulose microfibreuse à de l'eau et mélanger,
ajouter un tensioactif, puis mélanger,
ajouter des matières particulaires et après mélanger,
dans laquelle le système résultant est transparent et les matières particulaires sont en suspension dans celui-ci,
dans laquelle la cellulose microfibreuse est présente à une concentration allant d'environ 0,05 % à environ 1,0 % (p/p), et le tensioactif est présent à raison d'environ 51 % à environ 99 % (p/p tensioactif actif).

11. La méthode de la revendication 10 dans laquelle la cellulose microfibreuse est présente à raison d'environ 0,06 %.

12. La méthode de la revendication 10, dans laquelle la cellulose microfibreuse est présente à raison d'environ 0,075 %.

13. La méthode de la revendication 10, dans laquelle le tensioactif est présent à raison d'environ 80 % (p/p tensioactif actif).

14. La méthode de la revendication 10, dans laquelle le tensioactif est présent à raison d'environ 99 % (p/p tensioactif actif).

15. La méthode de n'importe lesquelles des revendications 10 à 14, dans laquelle la cellulose microfibreuse est préparée sous la forme d'un produit sec comprenant un co-agent.
